# EUROPEAN PATENT SPECIFICATION

(11) **EP 2 128 242 B1**
(45) Date of publication and mention of the grant of the patent: **16.05.2018**
(21) Application number: 08828063.1
(22) Date of filing: 30.09.2008
(51) Int. Cl.: C12M 1/00

(54) **CELL CULTURE MODULE**
ZELLKULTURMODUL
MODULE DE CULTURE CELLULAIRE

(30) Priority: 22.02.2008 JP 2008040916; 02.07.2008 JP 2008172949
(43) Date of publication of application: 02.12.2009
(73) Proprietor: CoorsTek KK, Tokyo 141-0032 (JP)
(72) Inventor: IMAIZUMI, Takafumi, Kanagawa 257-8566 (JP); KITAGAWA, Fumihiko, Kanagawa 257-8566 (JP); ITO, Madoka, Kanagawa 257-8566 (JP)
(74) Representative: Hoffmann Eitle
(86) International application number: PCT/JP2008/067703
(87) International publication number: WO 2009/104296

(56) References cited:
- WO-A1-93/18132
- WO-A2-2004/094586
- DE-A1- 10 249 903
- JP-A- 6 169 755
- JP-A- 2006 094 718
- JP-T- 2005 512 503
- JP-T- 2005 514 056
- US-A- 6 060 306
- US-B1- 6 576 458
- 'Dai 27 Kai The Annual Meeting of the Japanese Society for Biomaterials Yokoshu, 2005', article FUMIHIKO KITAGAWA ET AL.: 'Kokikoritsu Ceramics Saibo Baiyo Tantai no Kaihatsu', page 301, XP008116982

## Description

### Technical Field

The present invention relates to a cell culture module.

### Background Art

In recent years, with the advancement of the separation of stem cells from human bone marrow fluid, the differentiation and derivation to target tissue cells, a three-dimensional cultivation technique, the development of a scaffolding material, and the like, the tissue of a skin, bone, cartilage, blood vessel, heart valve, ligament, and the like, can be manufactured from the stem cells by cell culture. Some clinical applications have been started.

In such cell culture, there has been known a technique using a cell culture chip having cell culture cells which are orderly arranged on the surface of a substrate in array honeycomb shape, or the like, and agglutinate and hold specific cells (for example, Patent Document 1).

There has been known a cell culture device having a culturing case which includes a cell culture carrier and cells and stores a culture medium, and a magnetic field generator which generates a magnetic field for moving the cell culture carrier in the culture medium (for example, Patent Document 2) .

DE 102 49 903 A1 and US 6, 060, 306 describe cell culture devices in which cells are cultured in a culture liquid on a carrier and mechanical loads are applied to the cells either by deformation of the carrier or by applying hydraulic pressure on the cells. For both the purpose is to mimic physiological conditions and to culture mechanically robust cells.
Patent Document 1: JP-A KOKAI No. 2005-27598
Patent Document 2: JP-A KOKAI No. 2004-313007

### Disclosure of the Invention

### Problems to be Solved by the Invention

In the cell culture techniques described in Patent Documents 1 and 2, an operation which separates cultured cells from the cell culture chip or the cell culture carrier is necessary. The separating operation is usually performed using the enzyme treatment such as the trypsin treatment.

However, when such enzyme treatment is performed for a long time, the cultured cells can be deteriorated to some degree. The cells themselves can be dead. There is also a problem that the enzyme treatment such as the trypsin treatment is very complicated.

The present invention has been made in view of the above problems and an object of the present invention is to provide a cell culture module which can easily separate cells cultured in a cell culture carrier from the cell culture carrier without performing the enzyme treatment such as the trypsin treatment. Means for Solving the Problems

A cell culture module according to the present invention being the cell culture module according to claim 1.

With such structure, the cells cultured on the cell culture carrier can be easily separated from the cell culture carrier without performing the enzyme treatment such as the trypsin treatment.

A cell culture module according to the present invention preferably including:
a second culture liquid hydraulic pressure supplying portion which supplies a hydraulic pressure on the concave portion on the first surface of the cell culture carrier.

With such structure, the cells which resist the pressure of a cartilage cell, and the like, can be cultured and the cells cultured on the cell culture carrier can be easily separated from the cell culture carrier without performing the enzyme treatment such as the trypsin treatment.

A cell culture module according to the present invention preferably including:
a circulating portion which circulates the culture liquid filled in the space facing the first surface of the cell culture carrier and the space facing the second surface of the cell culture carrier.

With such structure, the cells which resist the pressure of a cartilage cell, and the like, can be cultured, and the cells cultured on the cell culture carrier can be easily separated from the cell culture carrier without performing the enzyme treatment such as the trypsin treatment.

A cell culture module according to the present invention including:
a cell culture carrier for culturing cells,
a sealing member sealably holding in an inner space the cell culture carrier which divides the inner space into a space facing a first surface of the cell culture carrier on one side and a space facing a second surface of the cell culture carrier on the other;
a culture liquid being filled in the space facing a first surface of the cell culture carrier and the space facing a second surface of the cell culture carrier,
characterized in that
the cell culture carrier is made up of a porous body and has a concave portion for culturing cells on the first surface, and a void of the porous body is communicated from the first surface to a second surface opposite to the first surface, and
the module further comprises
a circulating portion filling a culture liquid in the space facing the first surface of the cell culture carrier (S) and the space facing the second surface of the cell culture carrier, circulating the filled culture liquid, and supplying a hydraulic pressure onto the concave portion by a circulation,
a varying portion varying a circulating direction of the circulating portion, supplying a hydraulic pressure onto the second surface opposite to the first surface by a second circulation, and,
a cell recovering portion recovering the cells floating in the space facing a first surface of the cell culture carrier,
whereby the cells cultured on the concave portion are separated from the concave portion by the hydraulic pressure onto the second surface supplied by the varying portion and the cells floating in the space facing a first surface of the cell culture carrier are recovered by the cell recovering portion.

With such structure, the cells which resist the pressure of a cartilage cell, and the like, can be cultured, and the cells cultured on the cell culture carrier can be easily separated from the cell culture carrier without performing the enzyme treatment such as the trypsin treatment.

Preferably, the cell culture module further includes a second hydraulic pressure supplying portion which supplies a hydraulic pressure from the horizontal direction of the cell culture carrier held in the inner space.

With such structure, the cell culture module according to the present invention can perform perfusion culture.

Preferably, the average void diameter of a void of the porous body comprising the cell culture carrier is 10 nm or more and 10 µm or less and the void is communicated from the first surface to the second surface.

Preferably, the porous body constituting the cell culture carrier is made of ceramics of at least any one of zirconia, yttria, titania, alumina, silica, hydroxyapatite, and β-tricalcium phosphate or glass.

With such structure, the hydraulic pressure supplied onto the second surface of the cell culture carrier can be transmitted to the surface of the concave portion of the cell culture carrier. Therefore, the cells cultured in the concave portion of the cell culture carrier can be easily separated from the cell culture carrier.

### Effect of the Invention

The present invention provides the cell culture module which can easily separate the cultured cells from the cell culture carrier without performing the enzyme treatment such as the trypsin treatment.

### Brief Description of the Drawings

Fig. 1 is a concept diagram illustrating the section structure of a cell culture module according to a first embodiment;
Fig. 2 is a concept diagram of assistance in explaining an example of a cell culture method using the cell culture module according to the first embodiment;
Fig. 3 is a concept diagram of assistance in explaining an example of the cell culture method using the cell culture module according to the first embodiment;
Fig. 4 is a concept diagram illustrating the section structure of the cell culture module according to a modification of the first embodiment;
Fig. 5 is a concept diagram illustrating the section structure of a cell culture module according to a second embodiment;
Fig. 6 is a concept diagram of assistance in explaining an example of a cell culture method using the cell culture module according to the second embodiment;
Fig. 7 is a concept diagram illustrating the section structure of a cell culture module according to a third embodiment;
Fig. 8 is a concept diagram of assistance in explaining an example of a cell culture method using the cell culture module according to the third embodiment;
Fig. 9 is a concept diagram of assistance in explaining an example of the cell culture method using the cell culture module according to the third embodiment;
Fig. 10 is a concept diagram of assistance in explaining an example of the cell culture method using the cell culture module according to the third embodiment;
Fig. 11 is a concept diagram illustrating the section structure of a cell culture module according to a fourth embodiment;
Fig. 12 is a concept diagram illustrating the section structure of a cell culture module according to a fifth embodiment;
Fig. 13 is a concept diagram of assistance in explaining an example of a cell culture method using the cell culture module according to the fifth embodiment;
Fig. 14 is a concept diagram of assistance in explaining an example of the cell culture method using the cell culture module according to the fifth embodiment;
Fig. 15 is a concept diagram illustrating the section structure of a cell culture module according to a sixth embodiment;
Fig. 16 is a concept diagram illustrating the section structure of a cell culture module according to a modification of the sixth embodiment;
Fig. 17 is a concept diagram illustrating the section structure of a cell culture module according to a seventh embodiment;
Fig. 18 is a concept diagram of assistance in explaining an example of a cell culture method using the cell culture module according to the seventh embodiment; and
Fig. 19 is a concept diagram of assistance in explaining an example of the cell culture method using the cell culture module according to the seventh embodiment.

### Explanation of the Reference Numerals

1 Cell culture module
10 Sealing member
20 Culture liquid hydraulic pressure supplying portion
30 Cell recovering portion
40 Air pressure supplying portion
50 Second culture liquid hydraulic pressure supplying portion
100 Culture liquid circulation system
130 Feeder cell supplying portion

### Best Mode for carrying out the Invention

The present invention will be described below in detail with reference to the drawings.

### (First embodiment)

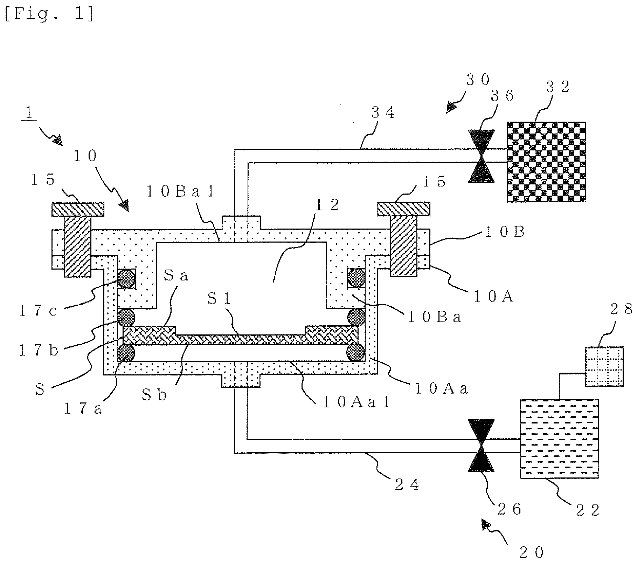

Fig. 1 is a concept diagram illustrating the section structure of a cell culture module according to a first embodiment.

As illustrated in Fig. 1, a cell culture module 1 according to the present embodiment comprises a sealing member 10, a culture liquid hydraulic pressure supplying portion 20, and a cell recovering portion 30.

For example, as illustrated in Fig. 1, the sealing member 10 comprises a lower cover 10A, an upper cover 10B provided on the lower cover 10A, and a fixing member 15 which fixes the lower cover 10A and the upper cover 10B.

As illustrated in Fig. 1, the lower cover 10A comprises a concave shape that has a concave portion 10Aa which holds a cell culture carrier S and stores a culture liquid.

As illustrated in Fig. 1, the upper cover 10B is provided on the lower cover 10A, forms an inner space 12 with the lower cover 10A, and has a convex portion 10Ba which sealably engages the concave portion 10Aa of the lower cover 10A so as to sealably hold the inner space 12.

As illustrated in Fig. 1, the holding of the cell culture carrier S, for example, arranges an O-ring 17a in a bottom portion 10Aa1 of the concave portion 10Aa of the lower cover 10A, the cell culture carrier S on the O-ring 17a, and an O-ring 17b on the cell culture carrier S. The O-ring 17a, the cell culture carrier S, and the O-ring 17b are thus arranged. The lower cover 10A and the upper cover 10B are fixed integrally. The O-ring 17b is pressed by the convex portion 10Ba of the upper cover 10B. The cell culture carrier S is held in the concave portion 10Aa of the lower cover 10A.

Before the concave portion 10Aa of the lower cover 10A engages the convex portion lOBa of the upper cover 10B, an O-ring 17c is fitted in a dent provided in the convex portion 10Ba of the upper cover 10B so as to sealably hold the inner space 12.

The lower cover 10A and the upper cover 10B, for example, are made of acrylic resin.

The fixing member 15, for example, is made of SUS304.

The O-rings 17a, 17b, and 17c, for example, are made of fluoro rubber.

As described above, in the present embodiment, the lower cover 10A and the upper cover 10B of the sealing member 10 are fixed integrally by the fixing member 15. The present invention is not limited to the above structure if the cell culture carrier S can be sealably held in the inner space 12.

The cell culture carrier S sealably held in the inner space 12 comprises a porous body having a concave portion S1 which cultures cells on its surface (a first surface) Sa.

The cell culture carrier S is made of ceramics of at least any one of zirconia, yttria, titania, alumina, silica, hydroxyapatite, and β-tricalcium phosphate or glass. The ceramics and glass are preferred due to their high biological stability.

The culture liquid hydraulic pressure supplying portion 20 is integrally or detachably provided in the bottom portion 10Aa1 of the lower cover 10A to supply the culture liquid into the inner space 12.

As illustrated in Fig. 1, the culture liquid hydraulic pressure supplying portion 20, for example, has a culture liquid storing portion 22 which stores the culture liquid, a culture liquid supply pipe 24 which has one end attached to the bottom portion 10Aa1 and the other end attached to the storing portion 22 and supplies the culture liquid stored in the storing portion 22 into the inner space 12, a supply amount controlling portion 26 which is attached to the supply pipe 24 and controls the supply amount of the culture liquid supplied into the inner space 12, and a supply pressure controlling portion 28 which is attached to the storing portion 22 and controls a supply pressure when the culture liquid is supplied.

The culture liquid storing portion 22, for example, comprises a resin storing case.

The culture liquid supply pipe 24, for example, comprises a resin tube.

The supply amount controlling portion 26, for example, comprises an electromagnetic valve which controls the supply amount of the culture liquid. The opening and closing operation and the opening and closing amount of the electromagnetic valve are controlled by a controlling portion, not illustrated. The supply amount of the culture liquid is controlled according to the opening and closing amount.

The supply pressure controlling portion 28, for example, comprises a high pressure gas supplying device which supplies a high pressure gas from the culture liquid storing portion 22 to the culture liquid supply pipe 24.

The culture liquid hydraulic pressure supplying portion 20 applies a liquid pressure in the direction of a back surface Sb of the cell culture carrier S held in the inner space 12 (a surface (a second surface) opposite to the surface (the first surface) Sa having the concave portion S1 of the cell culture carrier S) to supply a hydraulic pressure onto the second surface Sb.

To apply the hydraulic pressure, in the state that the culture liquid is filled in the sealed inner space 12, the supply amount controlling portion 26 and the supply pressure controlling portion 28 are controlled so as to supply the culture liquid into the inner space 12.

That is, in the state that the culture liquid is filled in the inner space 12, the supply amount controlling portion 26 is brought to the opened state. The supply pressure of the culture liquid is increased by the supply pressure controlling portion 28 to supply the large hydraulic pressure onto the second surface Sb of the cell culture carrier S. The supply pressure of the culture liquid is decreased by the supply pressure controlling portion 28 to supply the small hydraulic pressure onto the second surface Sb of the cell culture carrier S.

The hydraulic pressure is applied to the second surface Sb of the cell culture carrier S. The cells cultured in the concave portion S1 of the cell culture carrier S are separated from the cell culture carrier S. The cells are floated in the culture liquid filled in the inner space 12.

To transmit the hydraulic pressure supplied onto the second surface Sb of the cell culture carrier S to the surface of the concave portion S1 of the cell culture carrier S, the cell culture carrier S need to comprise a porous body.

More preferably, the average void diameter of a void of the porous body constituting the cell culture carrier S is 10 nm or more and 10 µm or less. Preferably, the void is communicated from the surface of the concave portion S1 of the cell culture carrier S to the second surface Sb.

With such structure, the hydraulic pressure supplied onto the second surface Sb of the cell culture carrier S can be transmitted to the surface of the concave portion S1 of the cell culture carrier S. The cells cultured in the concave portion S1 of the cell culture carrier S can be easily separated from the cell culture carrier S.

The cell recovering portion 30 is integrally or detachably provided in a top portion 10Ba1 of the upper cover 10B. The cells are cultured in the concave portion S1 of the cell culture carrier S. The cells separated from the cell culture carrier S are recovered together with the culture liquid from the inner space 12 of the sealing member 10 by the hydraulic pressure supplied onto the second surface Sb of the cell culture carrier S.

As illustrated in Fig. 1, the cell recovering portion 30, for example, comprises a cell storing portion 32 which stores the cultured cells together with the culture liquid, a cell discharge pipe 34 which has one end attached to the top portion 10Ba1 and the other end attached to the storing portion 32 and discharges the cells cultured in the inner space 12 together with the culture liquid from the inner space 12, and a discharge amount controlling portion 36 which is attached to the discharge pipe 34 and controls the discharge amount of the culture liquid discharged from the inner space 12.

The cell storing portion 32, for example, comprises a resin storing case.

The cell discharge pipe 34 comprises a resin tube.

The discharge amount controlling portion 36, for example, comprises an electromagnetic valve which controls the discharge amount. The opening and closing operation and the opening and closing amount of the electromagnetic valve are controlled by a controlling portion, not illustrated. The discharge amount is controlled according to the opening and closing amount.

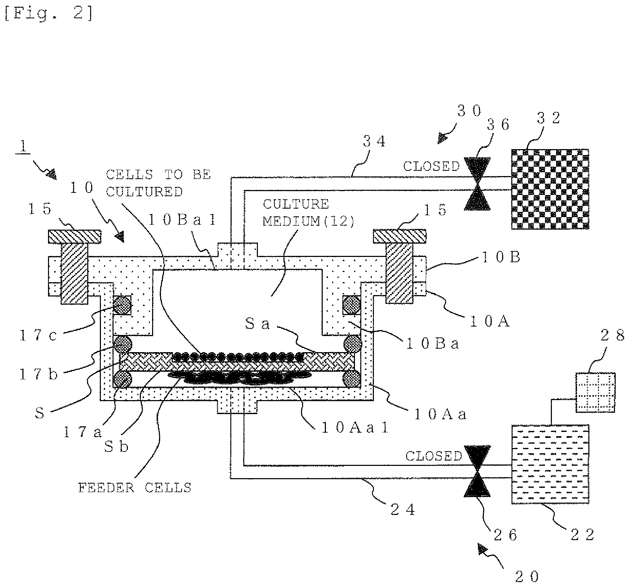

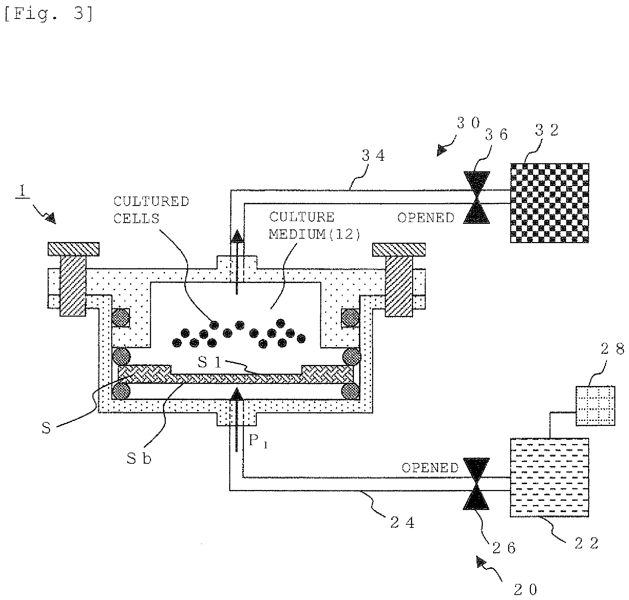

An example of a cell culture method using the cell culture module illustrated in Fig. 1 will be described using Figs. 2 and 3.

Figs. 2 and 3 are concept diagrams of assistance in explaining an example of the cell culture method using the cell culture module according to the present embodiment.

First, feeder cells are provided on the second surface Sb of the cell culture carrier S.

The lower cover 10A in concave shape is prepared. The O-ring 17a provided in the bottom portion 10Aa1 of the lower cover 10A, the cell culture carrier S on which the feeder cells are provided, and the O-ring 17b are stacked in that order so as to be provided in the concave portion 10Aa of the lower cover 10A. Cells to be cultured are provided in the concave portion S1 of the cell culture carrier S provided in the concave portion 10Aa of the lower cover 10A. The cells to be cultured are provided in the concave portion S1 of the cell culture carrier S. The provided cells adhere and held to the surface of the cell culture carrier S.

The O-ring 17c is inserted into the dent of the convex portion 10Ba of the upper cover 10B. The concave portion 10Aa of the lower cover 10A and the convex portion 10Ba of the upper cover 10B are respectively fitted engageably. The lower cover 10A and the upper cover 10B are fixed integrally by the fixing member 15. The sealing member 10 according to the present embodiment formed with the inner space 12 is structured.

Then, the culture liquid supply pipe 24 of the culture liquid hydraulic pressure supplying portion 20 is attached to the bottom portion 10Aa1 of the lower cover 10A. The cell discharge pipe 34 of the cell recovering portion 30 is attached to the top portion 10Ba1 of the upper cover 10B. The cell culture module 1 according to the present embodiment is structured.

Next, the discharge amount controlling portion 36 of the cell recovering portion 30 is brought to the "opened" state (in the present embodiment, the electromagnetic valve of the discharge amount controlling portion 36 is brought to the "opened" state). The supply amount controlling portion 26 of the culture liquid hydraulic pressure supplying portion 20 is brought to the "opened" state (in the present embodiment, the electromagnetic valve of the supply amount controlling portion 26 is brought to the "opened" state). Further, the supply pressure is applied from the supply pressure controlling portion 28. The culture liquid is supplied from the culture liquid storing portion 22 until the culture liquid is fully filled in the inner space 12.

When the culture liquid is fully filled in the inner space 12, the supply pressure from the supply pressure controlling portion 28 is stopped. The supply amount controlling portion 26 is brought to the "closed" state (in the present embodiment, the electromagnetic valve of the supply amount controlling portion 26 is brought to the "closed" state). The discharge amount controlling portion 36 of the cell recovering portion 30 is brought to the "closed" state at the same time (in the present embodiment, the electromagnetic valve of the discharge amount controlling portion 36 is brought to the "closed" state) . Hereby, the environment where the cells held in the concave portion S1 of the cell culture carrier S can be cultured, will be put into place (Fig. 2).

After the cells are cultured, the discharge amount controlling portion 36 of the cell recovering portion 30 is brought to the "opened" state to start the discharge of the culture liquid in the inner space 12. The supply amount controlling portion 26 of the culture liquid hydraulic pressure supplying portion 20 is brought to the "opened" state. Further, the supply pressure is applied from the supply pressure controlling portion 28 to newly start the supply of the culture liquid into the inner space 12. By this operation, a liquid pressure P₁ is supplied from the culture liquid hydraulic pressure supplying portion 20 in the direction of the second surface Sb of the cell culture carrier S. The hydraulic pressure is supplied onto the second surface Sb of the cell culture carrier S by the liquid pressure P₁. The cells cultured in the concave portion S1 of the cell culture carrier S are separated from the surface of the concave portion S1 of the cell culture carrier S. The cells are floated in the culture liquid in the inner space 12. The cells pass through the cell discharge pipe 34 of the cell recovering portion 30 together with the culture liquid. The cells are recovered in the cell storing portion 32 (Fig. 3).

As described above, the cell culture module according to the present embodiment has the above structure. The cells cultured on the cell culture carrier can be easily separated from the cell culture carrier without performing the enzyme treatment such as the trypsin treatment.

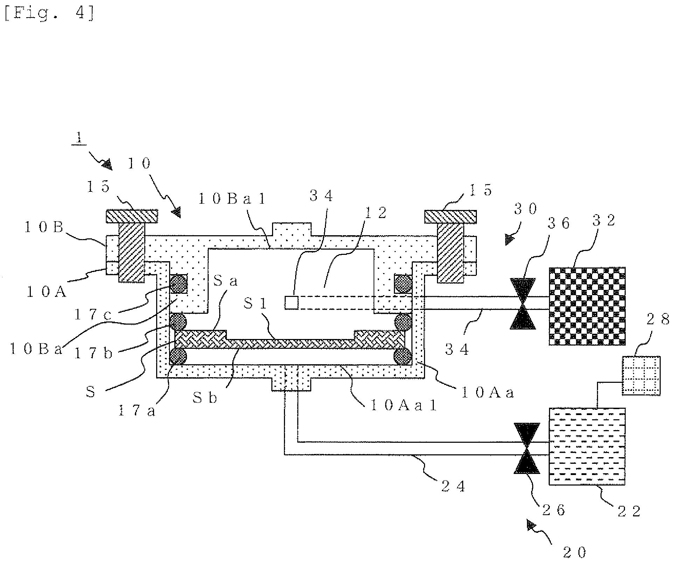

In Fig. 1, the cell recovering portion 30 described in the present embodiment is in the structure provided to the top portion 10Ba1 of the upper cover 10B, that is, above (in the vertical direction) the concave portion S1 of the cell culture carrier S. The present invention is not limited to this. For example, as illustrated in Fig. 4, the cell discharge pipe 34 of the cell recovering portion 30 may be provided in the horizontal direction (in the sheet surface direction in Fig. 4) vertically crossing the vertical direction of the cell culture carrier S held in the concave portion 10Aa of the lower cover 10A.

In the present embodiment, the controlling portions 26 and 36 using the electromagnetic valves are described. The controlling portions 26 and 36, for example, may be controlled by cylinders and the like, and comprise mere manual valves.

### (Second embodiment)

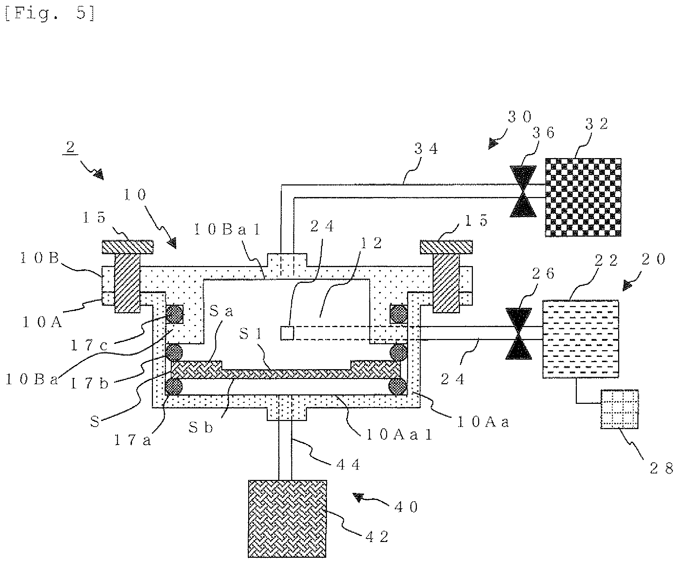

Fig. 5 is a concept diagram illustrating the section structure of a cell culture module according to a second embodiment.

In a cell culture module 2 according to the present embodiment, the culture liquid hydraulic pressure supplying portion 20 illustrated in Fig. 1 and described in the first embodiment is arranged in the horizontal direction (in the sheet surface direction in Fig. 5) of the cell culture carrier S. An air pressure supplying portion 40 is provided in the position of the culture liquid hydraulic pressure supplying portion 20 illustrated in Fig. 1. Except for this, the second embodiment is the same as the first embodiment and the description is omitted.

The air pressure supplying portion 40 is integrally or detachably provided in the bottom portion 10Aa1 of the lower cover 10A. An air pressure is supplied to the culture liquid filled in the inner space 12. The hydraulic pressure is supplied onto the second surface Sb of the cell culture carrier S.

The air pressure supplying portion 40, for example, comprises an air pressure generator 42 which generates the air pressure, and an air pressure supply pipe 44 which is integrally or detachably provided in the bottom portion 10Aa1 of the lower cover 10A and supplies the air pressure generated by the air pressure generator 42 into the inner space 12.

The air pressure generator 42, for example, comprises an air cylinder.

The air pressure supply pipe 44, for example, comprises a resin tube.

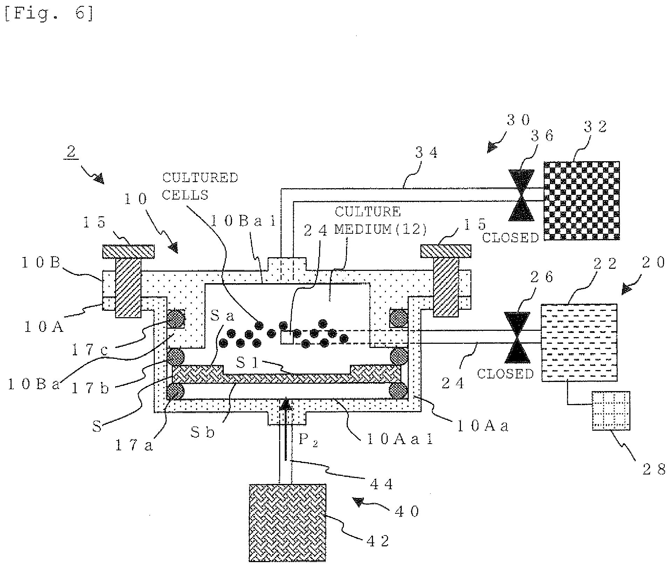

Next, an example of a cell culture method using the cell culture module illustrated in Fig. 5 will be described using Fig. 6. The description of the part overlapped with the part described in the first embodiment is omitted.

Fig. 6 is a concept diagram of assistance in explaining an example of the cell culture method using the cell culture module according to the present embodiment.

First, the sealing member 10 according to the present embodiment is structured. Then, the air pressure supply pipe 44 of the air pressure supplying portion 40 is attached to the bottom portion 10Aa1 of the lower cover 10A. The culture liquid supply pipe 24 of the culture liquid hydraulic pressure supplying portion 20 is attached in the horizontal direction of the cell culture carrier S. Further, the cell discharge pipe 34 of the cell recovering portion 30 is attached to the top portion 10Ba1 of the upper cover 10B. The cell culture module 2 according to the present embodiment is structured.

Then, the discharge amount controlling portion 36 of the cell recovering portion 30 is brought to the "opened" state. The supply amount controlling portion 26 of the culture liquid hydraulic pressure supplying portion 20 is brought to the "opened" state. Further, the supply pressure is applied from the supply pressure controlling portion 28. The culture liquid is supplied from the culture liquid storing portion 22 until the culture liquid is fully filled in the inner space 12.

When the culture liquid is fully filled in the inner space 12, the supply pressure from the supply pressure controlling portion 28 is stopped. The supply amount controlling portion 26 is brought to the "closed" state. The discharge amount controlling portion 36 of the cell recovering portion 30 is brought to the "closed" state at the same time. Hereby, the environment where the cells held in the concave portion S1 of the cell culture carrier S can be cultured, will be put into place.

After the cells are cultured, an air pressure P₂ by the air pressure generator 42 is supplied to the culture liquid filled in the direction of the second surface Sb of the cell culture carrier S held in the inner space 12. Hereby, the hydraulic pressure is supplied onto the second surface Sb of the cell culture carrier S. The cells cultured in the concave portion S1 of the cell culture carrier S are separated from the cell culture carrier S by the hydraulic pressure. The cells are floated in the culture liquid in the inner space 12 (Fig. 6) .

The supply amount controlling portion 26 of the culture liquid hydraulic pressure supplying portion 20 is brought to the "opened" state. Further, the supply pressure is applied from the supply pressure controlling portion 28 to newly start the supply of the culture liquid into the inner space 12. The discharge amount controlling portion 36 of the cell recovering portion 30 is brought to the "opened" state to start the discharge of the culture liquid in the inner space 12. The cultured cells floated in the inner space 12 pass through the cell discharge pipe 34 of the cell recovering portion 30 together with the culture liquid. The cells are recovered in the cell storing portion 32.

As described above, the cell culture module according to the present embodiment has the above structure. The cells cultured on the cell culture carrier can be easily separated from the cell culture carrier without performing the enzyme treatment such as the trypsin treatment.

In the cell culture module 2 according to the present embodiment, the culture liquid hydraulic pressure supplying portion 20 is arranged in the horizontal direction of the cell culture carrier S to supply the culture liquid into the inner space 12 from the horizontal direction. With the supply of the culture liquid, in the state that the culture liquid is filled in the inner space 12, the liquid pressure is applied to the inner space 12 to supply the hydraulic pressure from the horizontal direction.

The method of applying the liquid pressure is the same as that of the first embodiment and the description is omitted.

As described above, the hydraulic pressure is applied to the inner space 12 from the horizontal direction. The flow of the culture liquid in the inner space 12 can be controlled. The perfusion culture is enabled.

### (Third embodiment)

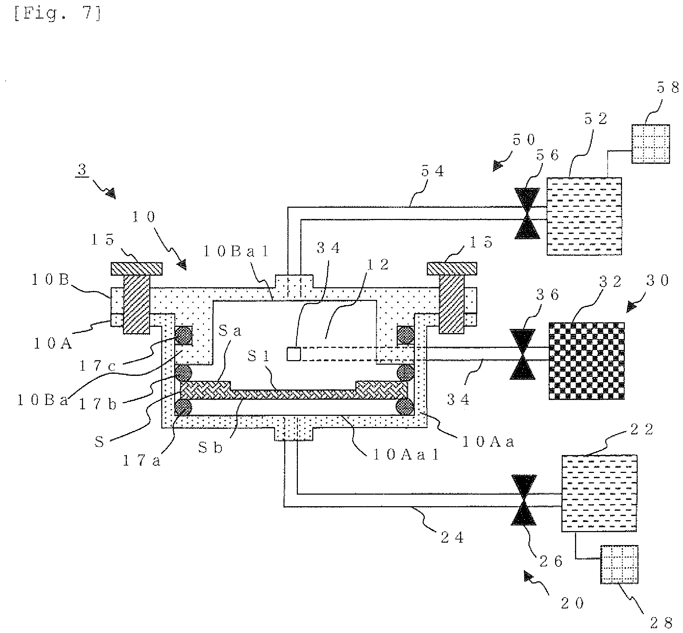

Fig. 7 is a concept diagram illustrating the section structure of a cell culture module according to a third embodiment.

In a cell culture module 3 according to the present embodiment, the point different from the first embodiment is that a second culture liquid hydraulic pressure supplying portion 50 which supplies the culture liquid into the inner space 12 is newly provided in the top portion 10Ba1 of the cell culture module illustrated in Fig. 4 and described in the first embodiment. Except for this, the third embodiment is the same as of the first embodiment and the description is omitted.

The second culture liquid hydraulic pressure supplying portion 50 is integrally or detachably provided in the top portion 10Ba1 of the upper cover 10B to supply the culture liquid into the inner space 12.

As illustrated in Fig. 7, the second culture liquid hydraulic pressure supplying portion 50 has a culture liquid storing portion 52 which stores the culture liquid, a culture liquid supply pipe 54 which has one end attached to the top portion 10Ba1 and the other end attached to the storing portion 52 and supplies the culture liquid stored in the storing portion 52 into the inner space 12, a supply amount controlling portion 56 which is attached to the supply pipe 54 and controls the supply amount of the culture liquid supplied into the inner space 12, and a supply pressure controlling portion 58 which is attached to the storing portion 52 and controls the supply pressure when the culture liquid is supplied.

The culture liquid storing portion 52, for example, comprises a resin storing case.

The culture liquid supply pipe 54, for example, comprises a resin tube.

The supply amount controlling portion 56, for example, comprises an electromagnetic valve which controls the supply amount of the culture liquid. The opening and closing operation and the opening and closing amount of the electromagnetic valve are controlled by a controlling portion, not illustrated. The supply amount of the culture liquid is controlled according to the opening and closing amount.

The supply pressure controlling portion 58, for example, comprises a high pressure gas supplying device which supplies a high pressure gas from the culture liquid storing portion 52 to the culture liquid supply pipe 54.

The second culture liquid hydraulic pressure supplying portion 50 applies the liquid pressure in the direction of the concave portion S1 of the cell culture carrier S held in the inner space 12 to supply the hydraulic pressure onto the concave portion S1.

To apply the hydraulic pressure, in the state that the culture liquid is filled in the sealed inner space 12, the supply amount controlling portion 56 and the supply pressure controlling portion 58 are controlled so as to supply the culture liquid into the inner space 12.

That is, in the state that the culture liquid is filled in the inner space 12, the supply amount controlling portion 56 is brought to the "opened" state. The supply pressure of the culture liquid is increased by the supply pressure controlling portion 58 to supply the large hydraulic pressure onto the concave portion S1 of the cell culture carrier S. The supply pressure of the culture liquid is decreased by the supply pressure controlling portion 58 to supply the small hydraulic pressure onto the concave portion Sl of the cell culture carrier S.

The liquid pressure is applied by the second culture liquid hydraulic pressure supplying portion 50 in the direction of the concave portion S1 of the cell culture carrier S. The cells are cultured in the concave portion S1. The cells which resist the pressure of a cartilage cell, and the like, can be cultured.

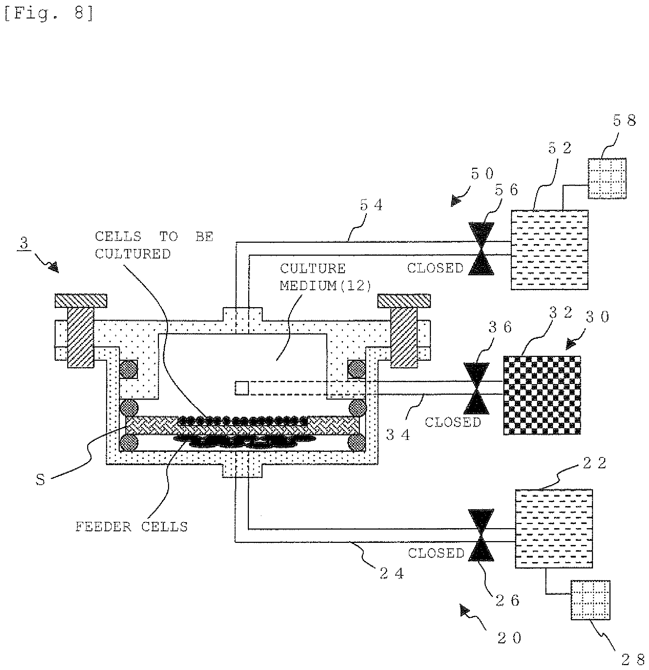

Next, a cell culture method using the cell culture module illustrated in Fig. 7 will be described using Figs. 8 to 10.

Figs. 8 to 10 are concept diagrams of assistance in explaining an example of the cell culture method using the cell culture module 3 according to the present embodiment. The description of the part overlapped with the part described in the first embodiment is omitted.

First, the cell culture module 3 according to the present embodiment is structured.

Next, the discharge amount controlling portion 36 of the cell recovering portion 30 is brought to the "opened" state. The supply amount controlling portion 26 of the culture liquid hydraulic pressure supplying portion 20 is brought to the "opened" state. Further, the supply pressure is applied from the supply pressure controlling portion 28. The culture liquid is supplied until the culture liquid is fully filled in the inner space 12.

In this case, similarly in the second culture liquid hydraulic pressure supplying portion 50, the supply amount controlling portion 56 and the supply pressure controlling portion 58 may be controlled to supply the culture liquid. The culture liquid may be supplied only by the second culture liquid hydraulic pressure supplying portion 50.

When the culture liquid is fully filled in the inner space 12, the supply pressure from the supply pressure controlling portion 28 is stopped. The supply amount controlling portion 26 is brought to the "closed" state. The supply of the culture liquid in the culture liquid hydraulic pressure supplying portion 20 is stopped. In this case, similarly the discharge amount controlling portion 36 of the cell recovering portion 30 is brought to the "closed" state (Fig. 8).

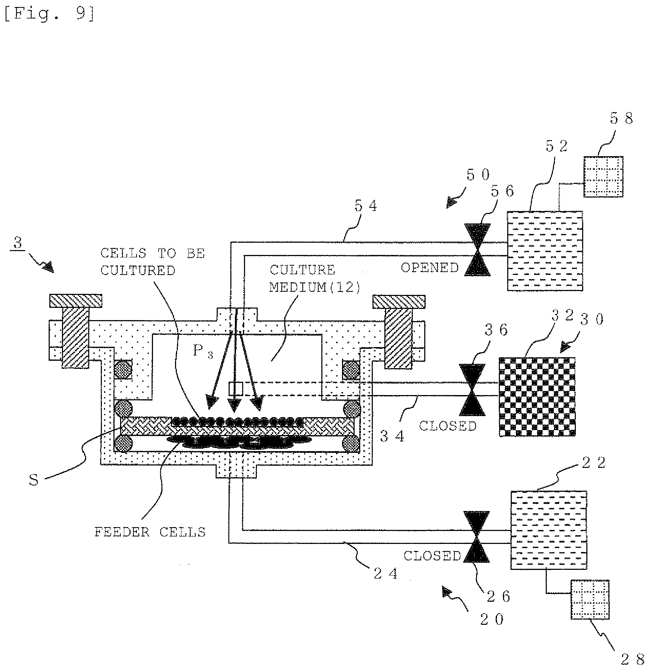

Next, the supply amount controlling portion 56 of the second culture liquid hydraulic pressure supplying portion 50 is brought to the "opened" state. The supply pressure is applied from the supply pressure controlling portion 58. A liquid pressure P₃ is supplied in the direction of the concave portion S1 of the cell culture carrier S to culture the cells (Fig. 9).

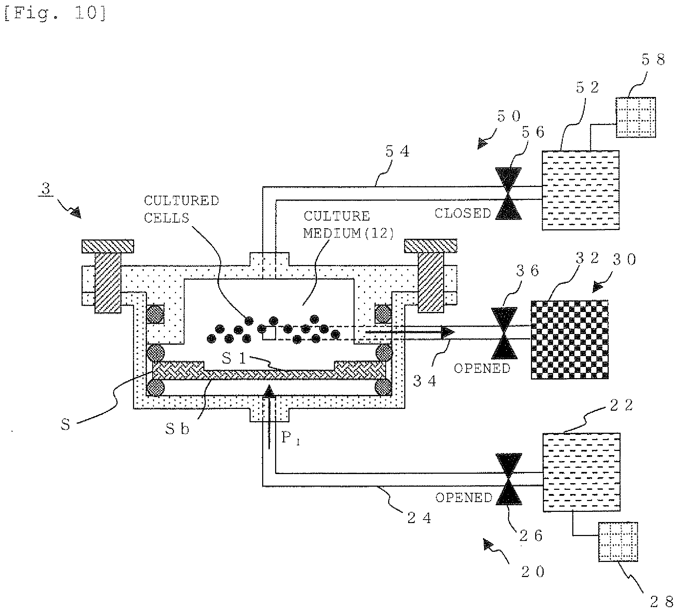

After the cells are cultured, the supply pressure from the supply pressure controlling portion 58 is stopped. The supply amount controlling portion 56 is brought to the "closed" state. The supply of the liquid pressure P₃ in the direction of the concave portion S1 of the cell culture carrier S is stopped. Then, the discharge amount controlling portion 36 of the cell recovering portion 30 is brought to the "opened" state to start the discharge of the culture liquid in the inner space 12. The supply amount controlling portion 26 of the culture liquid hydraulic pressure supplying portion 20 is brought to the "opened" state. Further, the supply pressure is applied from the supply pressure controlling portion 28 to newly start the supply of the culture liquid into the inner space 12. By this operation, the liquid pressure P₁ is supplied from the culture liquid hydraulic pressure supplying portion 20 in the direction of the second surface Sb of the cell culture carrier S. The hydraulic pressure is supplied onto the second surface Sb of the cell culture carrier S by the liquid pressure P₁. The cells cultured in the concave portion S1 of the cell culture carrier S are separated from the surface of the concave portion S1 of the cell culture carrier S. The cells are floated in the culture liquid in the inner space 12. The cells pass through the cell discharge pipe 34 of the cell recovering portion 30 together with the culture liquid. The cells are recovered in the cell storing portion 32 (Fig. 10).

As described above, the cell culture module according to the present embodiment has the above structure. The cells which resist the pressure of a cartilage cell, and the like, can be cultured. The cells cultured on the cell culture carrier can be easily separated from the cell culture carrier without performing the enzyme treatment such as the trypsin treatment.

The second culture liquid hydraulic pressure supplying portion 50 described in the present embodiment may comprise the air pressure supplying portion 40 illustrated in Fig. 5 and described in the second embodiment. In this case, the air pressure is supplied to the culture liquid filled in the inner space 12 by the air pressure supplying portion 40. The hydraulic pressure can be supplied onto the concave portion S1 of the cell culture carrier S. The same effect as that described in the present embodiment can be obtained.

### (Fourth embodiment)

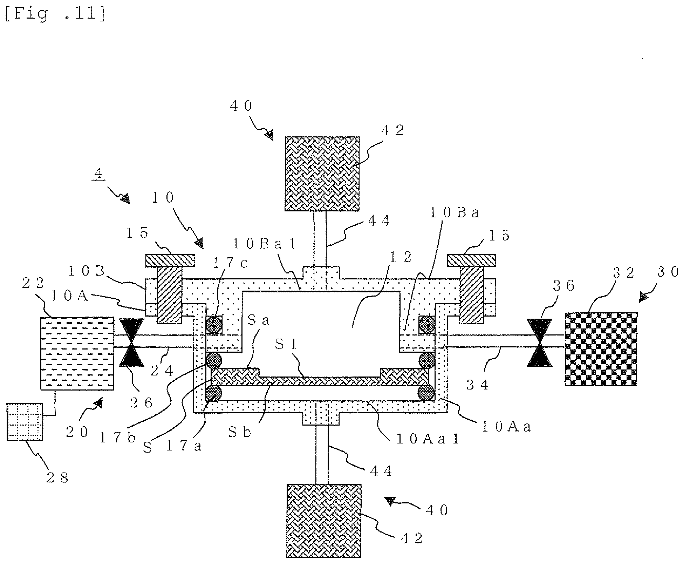

Fig. 11 is a concept diagram illustrating the section structure of a cell culture module according to a fourth embodiment.

In the cell culture module 4 according to the present embodiment, the point different from the third embodiment is that the culture liquid hydraulic pressure supplying portion 20 and the second culture liquid hydraulic pressure supplying portion 50 illustrated in Fig. 7 and described in the third embodiment are respectively replaced with the air pressure supplying portion 40 described in the second embodiment and the culture liquid hydraulic pressure supplying portion 20 is newly provided in the direction opposite to the cell recovering portion 30 provided in the horizontal direction of the cell culture carrier S. Except for this, the fourth embodiment is the same as the third embodiment and the description is omitted.

The air pressure supplying portion 40 attached to the bottom portion 10Aa1 of the lower cover 10A has the same structure and operation as those of the second embodiment and the description is omitted. The air pressure supplying portion 40 attached to the top portion Ba1 of the upper cover 10B has the same structure and operation as those of the third embodiment and the description is omitted.

Next, an example of a cell culture method using the cell culture module illustrated in Fig. 11 will be described.

The description of the part overlapped with the part described in the first to third embodiments is omitted.

First, a cell culture module 4 according to the present embodiment is structured.

Then, the discharge amount controlling portion 36 of the cell recovering portion 30 is brought to the "opened" state. Further, the supply amount controlling portion 26 of the culture liquid hydraulic pressure supplying portion 20 is brought to the "opened" state. The supply pressure is applied from the supply pressure controlling portion 28. The culture liquid is supplied from the culture liquid storing portion 22 until the culture liquid is fully filled in the inner space 12. When the culture liquid is fully filled in the inner space 12, the supply pressure from the supply pressure controlling portion 28 is stopped. The discharge amount controlling portion 36 of the cell recovering portion 30 and the supply amount controlling portion 26 of the culture liquid hydraulic pressure supplying portion 20 are brought to the "closed" state.

When the cells are cultured, the culturing is preformed while the hydraulic pressure is supplied onto the concave portion S1 of the cell culture carrier S by the air pressure supplying portion 40 attached to the top portion Ba1 of the upper cover 10B. Hereby, the cells which resist the pressure of a cartilage cell, and the like, can be cultured.

While the cells are cultured, the liquid pressure may be applied from the culture liquid hydraulic pressure supplying portion 20 into the inner space 12 in the horizontal direction of the cell culture carrier S. With such structure, the flow of the culture liquid in the inner space 12 can be controlled. The perfusion culture is enabled.

After the cells are cultured, the hydraulic pressure is supplied onto the second surface Sb of the cell culture carrier S by the air pressure supplying portion 40 attached to the bottom portion 10Aa1 of the lower cover 10A. The cells cultured in the concave portion S1 of the cell culture carrier S are separated from the cell culture carrier S. The cells are floated in the culture liquid in the inner space 12.

Then, the supply amount controlling portion 26 of the culture liquid supplying portion 20 is brought to the "opened" state. The supply pressure is applied from the supply pressure controlling portion 28 to newly start the supply of the culture liquid into the inner space 12. The discharge amount controlling portion 36 of the cell recovering portion 30 is brought to the "opened" state to start the discharge of the culture liquid in the inner space 12. The cultured cells floated in the inner space 12 pass through the cell discharge pipe 34 of the cell recovering portion 30 together with the culture liquid. The cells are recovered in the cell storing portion 32.

As described above, the cell culture module according to the present embodiment has the above structure. The cells which resist the pressure of a cartilage cell, and the like, can be cultured. The cells cultured on the cell culture carrier can be easily separated from the cell culture carrier without performing the enzyme treatment such as the trypsin treatment. Further, the culture liquid hydraulic pressure supplying portion 20 is provided in the direction opposite to the cell recovering portion 30 provided in the horizontal direction of the cell culture carrier S. It also has the effect that the speed which recovers the cultured cells in the cell recovering portion 32 can be improved.

### (Fifth embodiment)

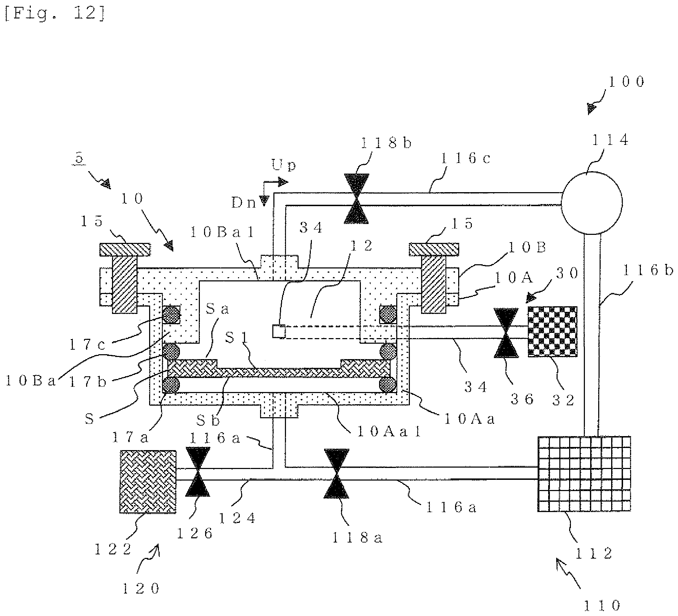

Fig. 12 is a concept diagram illustrating the section structure of a cell culture module according to a fifth embodiment.

In a cell culture module 5 according to the present embodiment, the point different from the first embodiment is that a culture liquid circulation system 100 is newly provided which circulates the culture liquid in the inner space 12 is replaced with the culture liquid hydraulic pressure supplying portion 20 illustrated in Fig. 4 and described in the first embodiment. Except for this, the fifth embodiment is the same as the first embodiment and the description is omitted.

As illustrated in Fig. 12, the culture liquid circulation system 100, for example, comprises a circulating portion 110 which circulates the culture liquid filled in the inner space 12, and a varying portion 120 which varies the circulating direction (Dn or Up) of the circulating portion 110.

The circulating portion 110 has a culture liquid storing portion 112 which previously holds the culture liquid and temporarily stores the circulated culture liquid, a supplying device 114 which supplies the culture liquid stored in the storing portion 112 into the inner space 12, pipes 116a, 116b, and 116c which couple the sealing member 10, the storing portion 112, and the supplying device 114, and circulation amount controlling portions 118a and 118b which are attached to the pipes 116a and 116c and control ON/OFF of the circulation of the circulating portion 110 and its circulation amount.

The varying portion 120 comprises an air pressure generator 122 which supplies the air pressure to the pipe 116a of the circulating portion 110, a pipe 124 which has one end integrally or detachably coupled to the pipe 116a and the other end connected to the generator 122, and an air pressure controlling portion 126 which is attached to the pipe 124, prevents the inclusion of the culture liquid from the pipe 116a into the generator 122, and controls ON/OFF of the air pressure.

The culture liquid storing portion 112, for example, comprises a resin storing case.

The supplying device 114, for example, comprises a pump.

The pipes 116a, 116b, and 116c, for example, comprise resin tubes.

The circulation amount controlling portions 118a and 118b, for example, comprise electromagnetic valves described in the first embodiment.

The air pressure generator 122, for example, comprises an air cylinder.

The pipe 124, for example, comprises a resin tube.

The air pressure controlling portion 126, for example, comprises an electromagnetic valve described in the first embodiment.

In the cell culture module 5 illustrated in the present embodiment, the opening and closing operation of the controlling portions 118a, 118b, and 126 are used fully to control the circulating direction (Dn, Up) between the sealing member 10 and the circulating portion 110. The opening and closing operation of the controlling portions 118a, 118b, and 126 is controlled such that the circulating direction is Dn. The culture liquid is circulated between the sealing member 10 and the circulating portion 110 to apply the liquid pressure in its circulating direction (Dn). The hydraulic pressure is supplied onto the concave portion S1 of the cell culture carrier S. The opening and closing operation of the controlling portions 118a, 118b, and 126 is controlled such that the circulating direction is Up. The culture liquid is circulated between the sealing member 10 and the circulating portion 110 to apply the liquid pressure in its circulating direction (Up) . The hydraulic pressure is supplied onto the second surface Sb of the cell culture carrier S. The control of the opening and closing operation of the controlling portions 118a, 118b, and 126 will be described later.

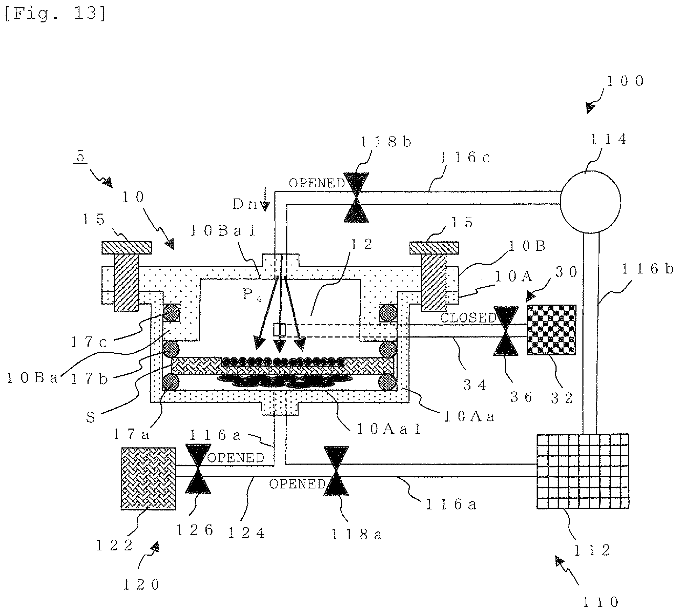

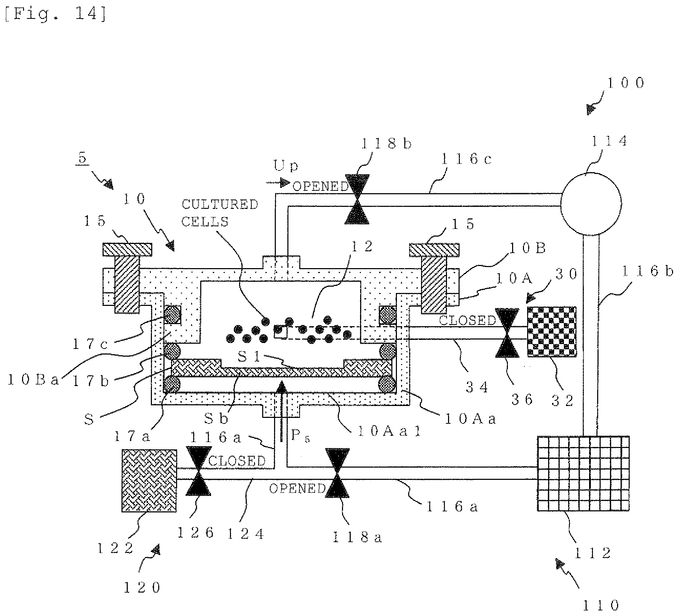

Next a cell culture method using the cell culture module illustrated in Fig. 12 will be described using Figs. 13 and 14.

Figs. 13 and 14 are concept diagrams of assistance in explaining an example of the cell culture method using the cell culture module according to the present embodiment. The description of the part overlapped with the part described in the first embodiment is omitted.

First, the cell culture module 5 according to the present embodiment is structured.

Next, the circulation amount controlling portions 118a and 118b are brought to the "opened" state. The discharge amount controlling portion 36 is brought to the "closed" state. The culture liquid is supplied from the culture liquid storing portion 112 which previously stores the culture liquid into the inner space 12 of the sealing member 10 using the supplying device 114 until the culture liquid is fully filled.

The direction supplying the culture liquid can be controlled by the opening and closing operation of the air pressure controlling portion 126. That is, the air pressure controlling portion 126 is brought to the "opened" state so that the supplying direction can be controlled to Dn. The air pressure controlling portion 126 is brought to the "closed" state so that the supplying direction can be controlled to Up.

Next, in the state that the culture liquid is fully filled in the inner space 12, the direction supplying the culture liquid is Dn. When the direction supplying the culture liquid into the inner space 12 is Dn, the supplying direction is not changed, thereby continuing the supply. When the direction supplying the culture liquid into the inner space 12 is Up, the supplying direction is changed to Dn, thereby continuing the supply. The supply of the culture liquid is continued. The culture liquid is circulated between the inner space 12 of the sealing member 10 and the circulating portion 110.

The direction supplying the culture liquid is Dn, thereby continuing the supply. The culture liquid is circulated between the sealing member 10 and the circulating portion 110. A liquid pressure P₄ is supplied in the direction of the concave portion S1 of the cell culture carrier S (Fig. 13). The cells which resist the pressure of a bone cell, and the like, can be cultured.

After the cells are cultured, the air pressure controlling portion 126 is brought to the "closed" state to change the supplying direction to Up.

As described above, the direction supplying the culture liquid is Up. A liquid pressure P₅ is supplied onto the second surface Sb of the cell culture carrier S. The cells cultured in the concave portion S1 of the cell culture carrier S are separated from the concave portion S1 of the cell culture carrier S by the liquid pressure. The cells are floated in the culture liquid in the inner space 12 (Fig. 14).

Then, the discharge amount controlling portion 36 is brought to the "opened" state. The controlling portion 118b is brought to the "closed" state. The cultured cells pass through the cell discharge pipe 34 of the cell recovering portion 30 together with the culture liquid. The cells are recovered in the cell recovering portion 32.

As described above, the cell culture module according to the present embodiment has the above structure . The cells which resist the pressure of a cartilage cell, and the like, can be cultured. The cells cultured on the cell culture carrier can be easily separated from the cell culture carrier without performing the enzyme treatment such as the trypsin treatment. The culture liquid can be circulated. The perfusion culture is enabled.

### (Sixth embodiment)

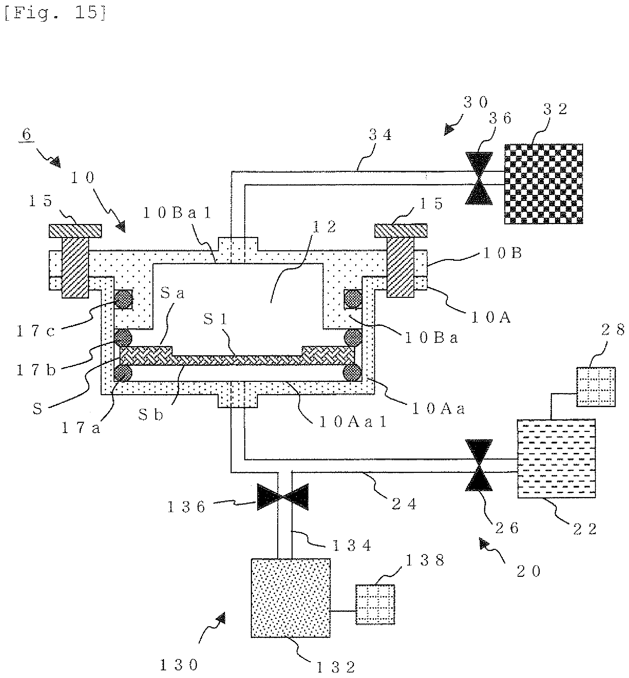

Fig. 15 is a concept diagram illustrating the section structure of a cell culture module according to a sixth embodiment.

In cell culture module 6 according to the present embodiment, the point different from the first embodiment is that the cell culture module 6 newly has a feeder cell supplying portion 130 which supplies the feeder cells to the culture liquid supply pipe 24 of the culture liquid supplying portion 20 described in the first embodiment. Except for this, the sixth embodiment is the same as the first embodiment and the description is omitted.

The feeder cell supplying portion 130 is integrally or detachably provided in the culture liquid supply pipe 24 to supply the feeder cells into the inner space 12 together with the culture liquid.

As illustrated in Fig. 15, the feeder cell supplying portion 130 comprises a cell storing portion 132 which stores the feeder cells and the culture liquid, a cell supply pipe 134 which has one end attached to the culture liquid supply pipe 24 and the other end attached to the storing portion 132 and supplies the feeder cells and the culture liquid stored in the storing portion 132 into the inner space 12, a supply amount controlling portion 136 which is attached to the supply pipe 134 and controls the supply amount of the feeder cells and the culture liquid supplied into the inner space 12, and a supply pressure controlling portion 138 which is attached to the storing portion 132 and controls the supply pressure when the feeder cells and the culture liquid are supplied.

The cell storing portion 132, for example, comprises a resin storing case.

The cell supply pipe 134, for example, comprises a resin tube.

The supply amount controlling portion 136, for example, comprises an electromagnetic valve as in the first embodiment.

The supply pressure controlling portion 138, for example, comprises a high pressure gas supplying device which supplies a high pressure gas from the cell storing portion 132 to the cell supply pipe 134.

In the method of supplying the feeder cells into the inner space 12, for example, when the culture liquid is supplied from the culture liquid hydraulic pressure supplying portion 20 into the inner space 12, the supply amount controlling portion 136 is brought to the "opened" state. The supply pressure is applied from the supply pressure controlling portion 138. The feeder cells and the culture liquid in the cell storing portion 132 are supplied at the same time. Hereby, the feeder cells are flowed in the culture liquid supply tube 24 together with the culture liquid. The cells adhere to the second surface Sb of the cell culture carrier S.

The cell culture module according to the present embodiment has such structure. The feeder cells need not to be provided on the second surface Sb of the cell culture carrier S by a different operation. The operation becomes efficient. The feeder cells can directly adhere to the second surface Sb of the cell culture carrier S in the cell culture module. The contamination of the cell culture can be prevented.

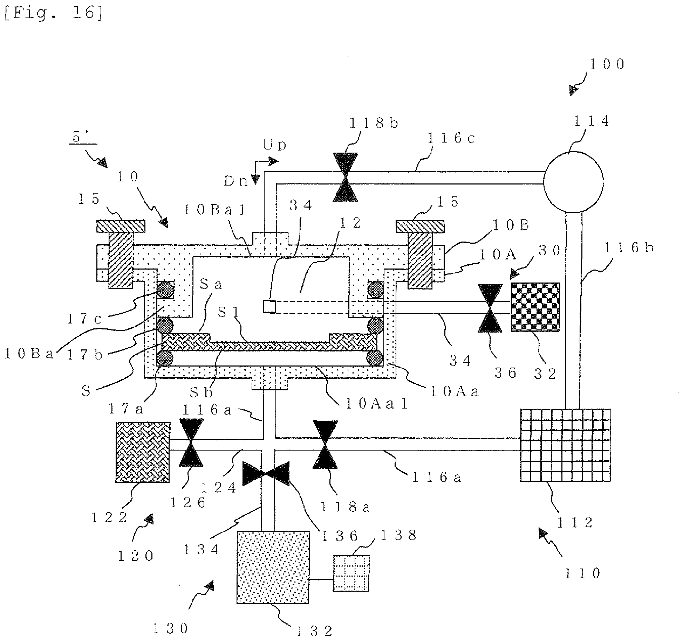

The feeder cell supplying portion 130 described in the present embodiment may be applied to the other above embodiments . When the feeder cell supplying portion 130 is applied to the cell culture module 5 according to the fifth embodiment, the cell culture module may have a structure as illustrated in Fig. 16.

### (Seventh embodiment)

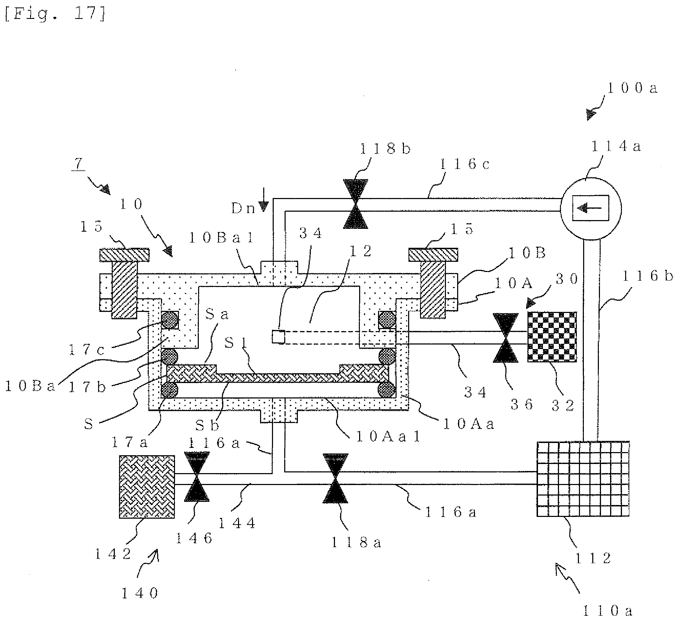

Fig. 17 is a concept diagram illustrating the section structure of a cell culture module according to a seventh embodiment.

In a cell culture module 7 according to the present embodiment, the point different from the fifth embodiment is that a culture liquid circulation system 100 described in the fifth embodiment is replaced with a culture liquid circulation system 100a. Except for this, the seventh embodiment is the same as the fifth embodiment and the description is omitted.

As illustrated in Fig. 17, the culture liquid circulation system 100a, for example, comprises a circulating portion 110a which circulates the culture liquid filled in the inner space 12, and an air pressure supplying portion 140 which supplies the air pressure to the culture liquid filled in the inner space 12 and supplies the hydraulic pressure onto the second surface Sb of the cell culture carrier S.

The circulating portion 110a has the culture liquid storing portion 112 which previously holds the culture liquid and temporarily stores the circulated culture liquid, a supplying device 114a which supplies the culture liquid stored in the storing portion 112 into the inner space 12 from the circulating direction Dn, the pipes 116a, 116b, and 116c which couple the sealing member 10, the storing portion 112, and the supplying device 114a, and the circulation amount controlling portions 118a and 118b which are attached to the pipes 116a and 116c and control ON/OFF of the circulation of the circulating portion 110a and its circulation amount.

The air pressure supplying portion 140, for example, comprises an air pressure generator 142 which generates the air pressure, a pipe 144 which has one end integrally or detachably coupled to the pipe 116a and the other end connected to the air pressure generator 142, and an air pressure controlling portion 146 which is attached to the pipe 144, prevents the inclusion of the culture liquid from the pipe 116a into the air pressure generator 142, and controls ON/OFF of the air pressure.

The culture liquid storing portion 112, for example, comprises a resin storing case.

The supplying device 114a, for example, comprises a pump.

The pipes 116a, 116b, and 116c, for example, comprise resin tubes.

The circulation amount controlling portions 118a and 118b, for example, comprise electromagnetic valves described in the first embodiment.

The air pressure generator 142, for example, comprises an air cylinder.

The pipe 144, for example, comprises a resin tube.

The air pressure controlling portion 146, for example, comprises an electromagnetic valve described in the first embodiment.

In the cell culture module 7 illustrated in the present embodiment, the culture liquid is filled in the inner space 12 by the supplying device 114a supplying the culture liquid into the inner space 12 from the circulating direction Dn. The filled culture liquid is circulated from the circulating direction Dn. The hydraulic pressure is supplied onto the concave portion S1 of the cell culture carrier S held in the inner space 12 by the circulation. The air pressure is supplied to the culture liquid filled in the inner space 12 by the air pressure supplying portion 140. The hydraulic pressure is supplied onto the second surface Sb of the cell culture carrier S.

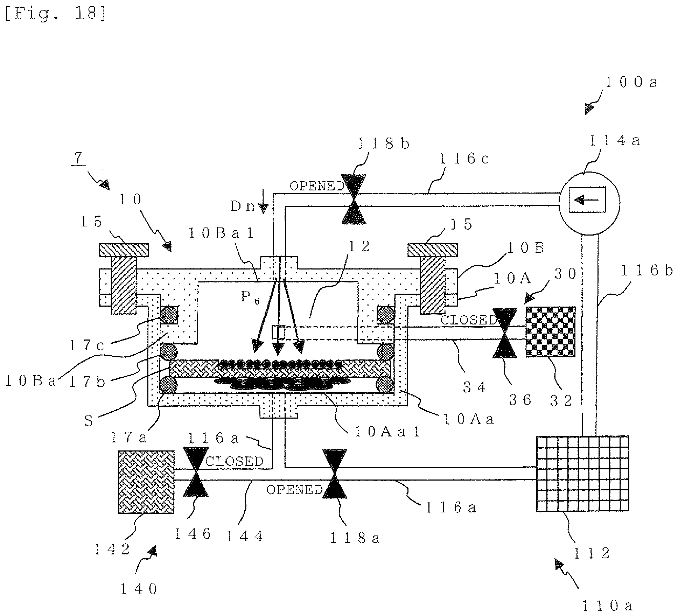

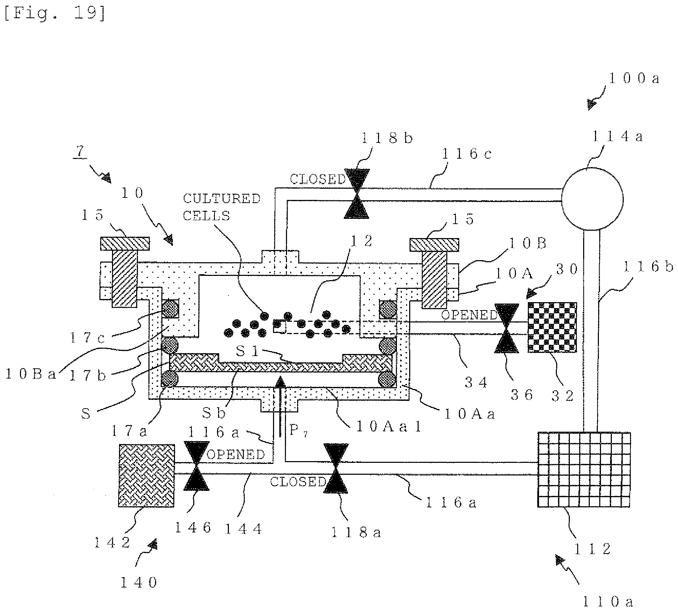

Next, a cell culture method using the cell culture module illustrated in Fig. 17 will be described using Figs. 18 and 19.

Figs. 18 and 19 are concept diagrams of assistance in explaining an example of the cell culture method using the cell culture module according to the present embodiment. The description of the part overlapped with the part described in the first embodiment is omitted.

First, the cell culture module 7 according to the present embodiment is structured.

Next, the circulation amount controlling portions 118a and 118b are brought to the "opened" state. The discharge amount controlling portion 36 is brought to the "closed" state. The air pressure controlling portion 146 is brought to the "closed" state. The culture liquid is supplied into the inner space 12 of the sealing member 10 from the circulating direction Dn using the supplying device 114a from the culture liquid storing portion 112 which previously stores the culture liquid.

In the state that the culture liquid is fully filled in the inner space 12, the supply is continued. The culture liquid is circulated between the inner space 12 of the sealing member 10 and the circulating portion 110a.

As described above, the direction supplying the culture liquid is Dn, thereby continuing the supply. While the cells are cultured, the culture liquid is circulated between the sealing member 10 and the circulating portion 110a. A liquid pressure P₆ is supplied in the direction of the concave portion S1 of the cell culture carrier S (Fig. 18). Therefore, the cells which resist the pressure of a bone cell, and the like, can be cultured.

After the cells are cultured, the circulation amount controlling portions 118a and 118b are brought to the "closed" state. The discharge amount controlling portion 36 is brought to the "closed" state. The air pressure controlling potion 146 is brought to the "opened" state. The air pressure is supplied to the culture liquid filled in the inner space 12. A liquid pressure P₇ is supplied onto the second surface Sb of the cell culture carrier S. By the liquid pressure, the cells cultured in the concave portion S1 of the cell culture carrier S are separated from the concave portion S1 of the cell culture carrier S. The cells are floated in the culture liquid in the inner space 12 (Fig. 19). Then, the discharge amount controlling portion 36 is brought to the "opened" state. The cultured cells pass through the cell discharge pipe 34 of the cell recovering portion 30. The cells are recovered in the cell storing portion 32.

As described above, the cell culture module according to the present embodiment has the above structure. Similar to the fifth embodiment, the cells which resist the pressure of a cartilage cell, and the like, can be cultured. The cells cultured on the cell culture carrier can be easily separated from the cell culture carrier without performing the enzyme treatment such as the trypsin treatment. Further, as compared with the fifth embodiment, the liquid pressure P₇ supplied onto the second surface Sb of the cell culture carrier S can be controlled at higher accuracy. The high precision control of the liquid pressure P₇ is enabled for each cell to be cultured.

### (Other embodiments)

The embodiments of the present invention are described above in detail.

For example, as described above, the cell culture modules 1 and 2 described in the first and second embodiments may further have the culture liquid circulation system 100 described in the fifth embodiment. In this case, as described in the fifth embodiment, the culture liquid circulation system 100 may be in the structure to supply the liquid pressure (hydraulic pressure) onto both the concave portion S1 and the second surface Sb. For example, the culture liquid circulation system 100 may be arranged so as to be opposite to the horizontal direction of the cell culture carrier S only for circulating the culture liquid in the inner space 12.

## Claims

1. A cell culture module comprising:
a cell culture carrier (S) for culturing cells,
a sealing member (10) sealably holding in an inner space (12) the cell culture carrier (S) which divides the inner space into a space facing a first surface (Sa) of the cell culture carrier (S) on one side and a space facing a second surface (Sb) of the cell culture carrier (S) on the other
**characterized in that**
the cell culture carrier (S) is made up of a porous body and made of ceramics of at least any one of zirconia, yttria, titania, alumina, silica, hydroxyapatite, and β-tricalcium phosphate or glass and has a concave portion (S1) for culturing cells on the first surface (Sa) of the cell culture carrier (S), and a void of the porous body with an averaged void diameter of 10 nm or more and 10 µm or less is communicated from the first surface (Sa) to a second surface (Sb) opposite to the first surface (Sa), and
the module further comprises:
a culture liquid hydraulic pressure supplying portion (20) supplying a hydraulic pressure onto the second surface (Sb) of the cell culture carrier (S) in a state that the culture liquid is filled in the space facing the first surface (Sa) of the cell culture carrier (S) and the space facing the second surface (Sb) of the cell culture carrier (S),
wherein the cells cultured on the concave portion (S1) are separated from the concave portion (S1) by being supplied the hydraulic pressure onto the second surface (Sb) by the culture liquid hydraulic pressure supplying portion (20); and
a cell recovering portion (30) recovering the cells that are separated from the concave portion (S1) by being supplied the hydraulic pressure onto the second surface (Sb) by the culture liquid hydraulic pressure supplying portion (20) and floating in the space facing the first surface (Sa) of the cell culture carrier (S), from the space facing a first surface (Sa).

2. A cell culture module according to claim 1, further comprising:
a second culture liquid hydraulic pressure supplying portion (20) which supplies a hydraulic pressure on the concave portion (S1) on the first surface (Sa) of the cell culture carrier (S).

3. The module according to claim 1, further comprising:
a circulating portion (110) which circulates the culture liquid filled in the space facing the first surface (Sa) of the cell culture carrier and the space facing the second surface (Sb) of the cell culture carrier (S).

## Patentansprüche

1. Zellkulturmodul, umfassend:
einen Zellkulturträger (S) zum Kultivieren von Zellen,
ein Abdichtelement (10), das in einem Innenraum (12) abdichtfähig den Zellkulturträger (S) hält, der den Innenraum in einen Raum, der einer ersten Oberfläche (Sa) des Zellkulturträgers (S) auf der einen Seite zugewandt ist und einen Raum, der einer zweiten Oberfläche (Sb) des Zellkulturträgers (S) auf der anderen zugewandt ist, aufteilt,
**dadurch gekennzeichnet, dass**
der Zellkulturträger (S) aus einem porösen Körper hergestellt ist und aus Keramik von mindestens einem von Zirkoniumoxid, Yttriumoxid, Titanoxid, Aluminiumoxid, Siliziumoxid, Hydroxyapatit und β-Tricalciumphosphat oder Glas besteht und einen konkaven Abschnitt (S1) zum Kultivieren von Zellen auf der ersten Oberfläche (Sa) des Zellkulturträgers (S) aufweist, und ein Hohlraum des porösen Körpers mit einem mittleren Hohlraumdurchmesser von 10 nm oder mehr und 10 µm oder weniger von der ersten Oberfläche (Sa) zu einer zweiten Oberfläche (Sb) entgegengesetzt zu der ersten Oberfläche (Sa) in Verbindung steht, und
das Modul weiter umfasst:
einen Abschnitt (20), der der Kulturflüssigkeit hydraulischen Druck zuführt, der einen hydraulischen Druck auf die zweite Oberfläche (Sb) des Zellkulturträgers (S) in einem Zustand zuführt, in dem die Kulturflüssigkeit in den Raum, der der ersten Oberfläche (Sa) des Zellkulturträgers (S) zugewandt ist und in den Raum, der der zweiten Oberfläche (Sb) des Zellkulturträgers (S) zugewandt ist, gefüllt ist,
wobei die auf dem konkaven Abschnitt (S1) kultivierten Zellen von dem konkaven Abschnitt (S1) durch Zuführen des hydraulischen Drucks auf die zweite Oberfläche (Sb) durch den Abschnitt (20), der der Kulturflüssigkeit hydraulischen Druck zuführt, getrennt werden; und
einen Zellgewinnungsabschnitt (30), der die Zellen gewinnt, die von dem konkaven Abschnitt (S1) durch Zuführen des hydraulischen Drucks auf die zweite Oberfläche (Sb) durch den Abschnitt (20), der der Kulturflüssigkeit hydraulischen Druck zuführt, getrennt werden und Schweben in den Raum, der der ersten Oberfläche (Sa) des Zellkulturträgers (S) zugewandt ist, aus dem Raum, der einer ersten Oberfläche (Sa) zugewandt ist.

2. Zellkulturmodul nach Anspruch 1, weiter umfassend:
einen zweiten Abschnitt (20), der der Kulturflüssigkeit hydraulischen Druck zuführt, der einen hydraulischen Druck dem konkaven Abschnitt (S1) auf der ersten Oberfläche (Sa) des Zellkulturträgers (S) zuführt.

3. Modul nach Anspruch 1, weiter umfassend:
einen zirkulierenden Abschnitt (110), der die in den der ersten Oberfläche (Sa) des Zellkulturträgers zugewandten Raum und den der zweiten Oberfläche (Sb) des Zellkulturträgers (S) zugewandten Raum gefüllte Kulturflüssigkeit im Kreislauf führt.

## Revendications

1. Module de culture cellulaire comprenant :
un support de culture cellulaire (S) pour cultiver des cellules,
un élément d'étanchéité (10) contenant de manière étanche dans un espace interne (12) le support de culture cellulaire (S) qui divise l'espace interne en un espace orienté vers une première surface (Sa) du support de culture cellulaire (S) d'un côté et en un espace orienté vers une seconde surface (Sb) du support de culture cellulaire (S) de l'autre
**caractérisé en ce que**
le support de culture cellulaire (S) est composé d'un corps poreux et fait d'une céramique d'au moins l'un quelconque parmi la zircone, l'oxyde d'yttrium, l'oxyde de titane, l'alumine, la silice, l'hydroxyapatite, et le phosphate tricalcique β ou d'un verre et présente une partie concave (S1) pour cultiver des cellules sur la première surface (Sa) du support de culture cellulaire (S), et un vide du corps poreux ayant un diamètre moyen de vide de 10 nm ou plus et de 10 µm ou moins est communiqué depuis la première surface (Sa) vers une seconde surface (Sb) opposée à la première surface (Sa), et
le module comprend en outre :
une partie de fourniture de pression hydraulique de liquide de culture (20) fournissant une pression hydraulique sur la seconde surface (Sb) du support de culture cellulaire (S) dans un état où le liquide de culture est rempli dans l'espace orienté vers la première surface (Sa) du support de culture cellulaire (S) et l'espace orienté vers la seconde surface (Sb) du support de culture cellulaire (S),
dans lequel les cellules mises en culture sur la partie concave (S1) sont séparées de la partie concave (S1) en fournissant la pression hydraulique sur la seconde surface (Sb) par la partie de fourniture de pression hydraulique de liquide de culture (20) ; et
une partie de récupération de cellules (30) récupérant les cellules qui sont séparées de la partie concave (S1) en fournissant la pression hydraulique sur la seconde surface (Sb) par la partie de fourniture de pression hydraulique de liquide de culture (20) et en flottant dans l'espace orienté vers la première surface (Sa) du support de culture cellulaire (S), depuis l'espace orienté vers une première surface (Sa).

2. Module de culture cellulaire selon la revendication 1, comprenant en outre :
une seconde partie de fourniture de pression hydraulique de liquide de culture (20) qui fournit une pression hydraulique sur la partie concave (S1) sur la première surface (Sa) du support de culture cellulaire (S).

3. Module selon la revendication 1, comprenant en outre :
une partie de circulation (110) qui fait circuler le liquide de culture rempli dans l'espace orienté vers la première surface (Sa) du support de culture cellulaire et l'espace orienté vers la seconde surface (Sb) du support de culture cellulaire (S).
